Europäisches Patentamt

European Patent Office

Office européen des brevets

(11) Veröffentlichungsnummer: **0 044 428**
**B1**

(12) **EUROPÄISCHE PATENTSCHRIFT**

(45) Veröffentlichungstag der Patentschrift:
21.09.83

(51) Int. Cl.³: **C 12 F 3/10, C 12 P 7/06**

(21) Anmeldenummer: 81104828.9

(22) Anmeldetag: 23.06.81

(54) **Verfahren zum Gewinnen einer konzentrierten Schlempe bei der Herstellung von Alkohol aus Stärke oder stärkehaltigen Rohstoffen.**

(30) Priorität: 26.06.80 DE 3023874

(43) Veröffentlichungstag der Anmeldung:
27.01.82 Patentblatt 82/4

(45) Bekanntmachung des Hinweises auf die Patenterteilung:
21.09.83 Patentblatt 83/38

(84) Benannte Vertragsstaaten:
AT BE CH DE FR GB IT LI LU NL SE

(56) Entgegenhaltungen:
FR-A-2 442 887
GB-A-1 109 311
GB-A-1 295 982
US-A-2 663 718

(73) Patentinhaber: Supraton F.J. Zucker GmbH, Am Henselsgraben 5, D-4040 Neuss 21 (DE)

(72) Erfinder: Zucker, Friedrich J., Dipl.-Ing., St. Andreas-Strasse 16, D-4040 Neuss 21 (DE)
Erfinder: Osthaus, Georg, Dipl.-Ing., Hardenbergstrasse 23, D-4040 Neuss 21 (DE)

(74) Vertreter: Keller, Johanna Carola et al, Deichmannhaus am Hauptbahnhof, D-5000 Köln 1 (DE)

# Verfahren zum Gewinnen einer konzentrierten Schlempe bei der Herstellung von Alkohol aus Stärke oder stärkehaltigen Rohstoffen

Ein bekanntes Problem der äthanolischen Gärung sind die geringen Substratkonzentrationen beim Gärvorgang, wodurch bei der bisher üblichen Arbeitsweise eine Schlempe mit nur etwa bis zu 8% Trockensubstanz gewonnen werden kann. Diese Trockensubstanz enthält bekanntlich als wesentliche Bestandteile neben Restzucker vor allem Aminosäuren, Proteine und Salze, und aufgrund dieser Tatsache haben die Schlempen, und zwar vor allem die Kartoffel- und Getreideschlempen, einen hohen Futterwert, der ohne Zweifel noch höher wäre, wenn der Anteil der Schlempe an Trockensubstanz erhöht werden könnte. Theoretisch müsste dies durch mehrmaliges Rückführen der Schlempe bei der alkoholischen Gärung zu erreichen sein, wobei bei mehrfacher Rückführung eine entsprechende Steigerung um ein Mehrfaches möglich sein müsste. In der Praxis hat sich jedoch gezeigt, dass eine mehrmalige Rückführung der Schlempe nicht möglich ist, da durch den thermischen Aufschluss der Stärke bzw. der stärkehaltigen Rohstoffe Nebenprodukte gebildet werden, die die anschliessende Gärung mittels Hefen beeinträchtigt. Hierbei handelt es sich beispielsweise um die Produkte der Maillard-Reaktion zwischen Proteinen und Kohlenhydraten.

Bekanntlich muss Stärke als nicht direkt vergärbares Kohlenhydrat mit Hilfe von Enzymen zunächst zu den vergärbaren Zuckern abgebaut werden, bevor die alkoholische Gärung möglich ist. Dazu ist es zunächst erforderlich, die stärkehaltigen Rohstoffe aufzuschliessen, wobei die Stärkezellen aus ihren Zellverbänden gelöst und geöffnet werden und die Stärke freigelegt wird, die dann leichter verkleistert und verzuckert werden kann. Dieser Aufschluss erfolgt beim sogenannten Dämpfverfahren meist im HENZE-Dampfer bei höheren Temperaturen unter Druck, worauf dann entspannt und gekühlt und anschliessend Enzym zugegeben wird, um die aufgeschlossene und verkleisterte Stärke zu verzukkern. Es handelt sich hierbei um ein nichtkontinuierliches, chargenweise durchzuführendes Verfahren, bei dem der Stärke-Rohstoff bei bis zu 150°C ca. 1½ bis 2 Stunden zu seiner Verkleisterung benötigt. Neben dem HENZE-Dampfer werden teilweise auch Düsenkocher eingesetzt, doch ist auch bei diesen die Temperatur relativ hoch, und zwar liegt sie im allgemeinen im Bereich zwischen 105°C und 115°C.

Ein Verfahren zum Verkleistern von Stärke bzw. stärkehaltigen Produkten unter erheblicher Energieeinsparung beschreibt darüber hinaus die DE-A-19 22 932, wobei Dampf und Stärkesuspension koaxial in das Zentrum einer Homogenisiermaschine eingeleitet und das Verkleistern der Stärke in einer Zone hoher Dichte von Scher- und Kavitationskräften durchgeführt wird. Bei diesem bekannten Verfahren kann mit Erfolg eine Homogenisiermaschine verwendet werden, die aus einem Gehäuse mit darin umlaufendem kegelstumpfförmigem Rotor besteht, dessen Mantelfläche mit koaxialen Ringen gestaffelten Durchmessers ausgestattet ist, die jeweils auf Lücke stehen mit gleichartigen Ringen an der dem Rotor gegenüberliegenden Gehäuseinnenwand. Auch bei diesem Verfahren wird noch mit vergleichsweise hohen Temperaturen gearbeitet, wenn diese auch niedriger sind als die beim HENZE-Dampfer und beim Düsenkocher üblichen Temperaturen.

Der Erfindung lag die Aufgabe zugrunde, ein Verfahren zur Verfügung zu stellen, mit dem es gelingt, bei der Herstellung von Alkohol aus Stärke oder stärkehaltigen Rohstoffen die Menge der gärungshemmenden Produkte wie der Maillard-Verbindungen in einem solchen Umfange zu reduzieren, dass eine konzentrierte Schlempe dadurch gewonnen werden kann, dass sie mehrmals rückgeführt und dabei ihr Trockenanteil erhöht werden kann.

Die Lösung dieser Aufgabe ist ein Verfahren zum Gewinnen einer konzentrierten Schlempe bei der Herstellung von Alkohol aus Stärke oder stärkehaltigen Rohstoffen, das dadurch gekennzeichnet ist, dass man Stärke oder stärkehaltige Rohstoffe in zweckmässig zerkleinerter Form zusammen mit für die Alkoholgewinnung geeigneten Enzymen unter gleichzeitigem Zuführen von Dampf in das Zentrum einer Homogenisiermaschine koaxial einführt und dort unter feinster Verteilung des Enzyms bei Temperaturen nicht unter 50°C und nicht über 115°C schlagartig mechanisch und thermisch verkleistert, den Stärkekleister nach dem Verflüssigen bis zur Glukose abbaut und die hierbei erhaltene Maische so vergärt, dass die mit reduzierter Trockensubstanz anfallende Schlempe nach Entfernen lediglich der Grobverunreinigungen durch beispielsweise Zentrifugieren ohne weitere Reinigung mehrfach zurückgeführt und als Prozesswasser wieder verwendet wird, bis der Trockensubstanzanteil der Schlempe bis zu 30% TS angestiegen ist. Vorzugsweise verkleistert man bei Temperaturen zwischen 85°C und 95°C.

Als Rohstoffe können bei diesem Verfahren neben Kartoffeln, Getreide, insbesondere Roggen, Weizen, Mais, Reis oder Hirse, auch die stärkehaltigen Wurzeln des Manihotstrauches Verwendung finden.

Als Homogenisiermaschine kann bei diesem Verfahren vorzugsweise eine Vorrichtung der oben geschilderten Art eingesetzt werden, die beispielsweise auch in der US-PS 3 995 838 beschrieben ist und aus einem Gehäuse mit darin umlaufendem kegelstumpfförmigem Rotor besteht, dessen Mantelfläche mit koaxialen Ringen gestaffelten Durchmessers ausgestattet ist, die jeweils auf Lücke stehen mit gleichartigen Ringen an der dem Rotor gegenüberliegenden Gehäuseinnenwand.

Der besondere Vorteil des Verfahrens gemäss der Erfindung besteht darin, dass lediglich bei Temperaturen um 70°C bis 90°C bei der Verkleisterung der stärkehaltigen Rohstoffe gearbeitet wird und dass bei diesen vergleichsweise niedrigen Temperaturen nur sehr geringe Mengen an Produkten der Maillard-Reaktion entstehen.

Ein weiterer wesentlicher Vorteil des Verfahrens gemäss der Erfindung besteht darin, dass es bei Stärkekonzentrationen bis zu 40% TS durchgeführt werden kann. Auf diese Weise ist es möglich, beim Einsatz frischer Knollen oder Wurzeln, die im allgemeinen ca. 40% TS enthalten, mindestens einen Teil der Schlempe als Verdünnungswasser einzusetzen. Werden als stärkehaltige Rohstoffe die obengenannten Getreidearten verwendet, so wird die Schlempe als sogenanntes Anlösewasser benutzt.

Erfindungsgemäss kann die Schlempe mehrfach, nämlich ca. 3- bis 4mal zurückgeführt und als Prozesswasser wieder verwendet werden. Da der Trockensubstanzanteil hierbei auf etwa 30% TS ansteigt, braucht bei der späteren Verwendung der konzentrierten Schlempe als Futtermittel eine weitaus geringere Menge Flüssigkeit als dies bisher notwendig gewesen ist, verdampft zu werden. Hinzu kommt, dass die Schlempemenge als solche auf etwa ein Viertel der bisher üblicherweise anfallenden Menge herabgesetzt und dabei gleichzeitig auch zusätzliche Abwasserprobleme gelöst werden. Ein besonderer Vorteil des Verfahrens gemäss der Erfindung besteht aber darin, dass durch die mehrfache Rückführung der Schlempe nicht so weit wie bisher heruntervergoren werden muss, da ja der in der Schlempe enthaltene Restzucker mehrfach wieder in den Gäransatz zurückgeht und dort ebenfalls vergoren wird.

Die Erfindung wird durch das nachstehende Beispiel weiter erläutert.

Beispiel

Aus 40 kg Getreide (mit ca. 12% Wasser) oder Tapiokamehl (mit ca. 12% Wasser) wird ein Ausgangsgemisch (mit ca. 35% TS) angesetzt und in das Zentrum einer Homogenisiermaschine koaxial eingeführt, wobei gleichzeitig Dampf zugeführt wird. Nach dem Verkleistern bei ca. 70°C wird die Stärke enzymatisch verflüssigt, danach mit Wasser 1:1 verdünnt und enzymatisch verzuckert (man hat etwa 200 l süsse Maische mit annähernd 17% TS), danach wird in an sich bekannter Weise vergoren.

Die bei der Destillation des Gärproduktes anfallende Schlempe enthält ca. 11% TS. Sie wird durch Zentrifugieren von verunreinigenden Grobstoffen, wie z.B. Hefezellen, befreit, wobei etwa 190 kg Schlempe mit etwa 9% TS erhalten werden.

160 kg dieser 9% TS enthaltenden Schlempe wird erneut mit 40 kg Rohstoff angesetzt und in der beschriebenen Weise wiederum verkleistert und vergoren und nach der Destillation von den Grobstoffen durch Zentrifugieren befreit. Hierbei werden wiederum etwa 190 kg Schlempe gewonnen, die nunmehr 17% TS enthalten. Beim Vermischen mit den verbliebenen 30 kg Schlempe mit 9% TS aus dem vorherigen Einsatz werden somit insgesamt 220 kg Schlempe mit ca. 16% TS erhalten. Auf diese Weise gelingt es bereits bei einmaliger Schlemperückführung, das für die Prozessführung erforderliche Frischwasser um 100%, die Gesamtmenge der anfallenden Schlempe um 27% und die für das Verdampfen erforderlichen Kosten um 45% zu reduzieren.

Insgesamt ist es erfindungsgemäss möglich, das Prozesswasser mindestens 3- bis 4mal im Kreislauf zu führen. Selbstverständlich ist es auch möglich, nur einen Teil der Schlempe als Verdünnungs- und Kühlwasser der konzentrierten Maische zu verwenden, doch sind hierbei die Vorteile geringer als oben angegeben.

## Patentansprüche

1. Verfahren zum Gewinnen einer konzentrierten Schlempe bei der Herstellung von Alkohol aus Stärke oder stärkehaltigen Rohstoffen, dadurch gekennzeichnet, dass man Stärke oder stärkehaltige Rohstoffe in zweckmässig zerkleinerter Form zusammen mit für die Alkoholgewinnung geeigneten Enyzmen unter gleichzeitigem Zuführen von Dampf in das Zentrum einer Homogenisiermaschine koaxial einführt und dort unter feinster Verteilung des Enzyms bei Temperaturen nicht unter 50°C und nicht über 115°C schlagartig mechanisch und thermisch verkleistert, den Stärkekleister nach dem Verflüssigen bis zur Glukose abbaut und die hierbei erhaltene Maische so vergärt, dass die mit reduzierter Trockensubstanz anfallende Schlempe nach Entfernen lediglich der Grobverunreinigungen durch beispielsweise Zentrifugieren ohne weitere Reinigung mehrfach zurückgeführt und als Prozesswasser wieder verwendet wird, bis der Trockensubstanzanteil der Schlempe bis zu 30% TS angestiegen ist.

2. Verfahren nach Anspruch 1, dadurch gekennzeichnet, dass man bei Temperaturen zwischen 85°C und 95°C verkleistert.

## Claims

1. A process for the recovery of a concentrated stillage in producing alcohol from starch or starch-containing raw materials, characterized in that starch or starch-containing raw materials, in a desirably comminuted form, are coaxially introduced together with enzymes which are suitable for the recovery of alcohol and with the simultaneous supply of steam into the center of a homogenizer where they are suddenly gelatinized mechanically and thermally with ultrafine division of the enzyme at temperatures not less than 50°C and not more than 115°C; the starch paste after liquefaction is degraded to the glucose and the resultant mash is fermented in such a manner that the stillage obtained with reduced dry substance, after removal of merely the coarse contaminations by, for example, centrifuging, is repeatedly returned without further purification and re-used as process liquor until the dry substance portion of the stillage has increased to 30% of dry substance.

2. A process according to claim 1, characterized in that said gelatinization is effected at temperatures between 85°C and 95°C.

### Revendications

1. Procédé d'obtention d'une vinasse concentrée pendant la production d'alcool à partir de l'amidon ou de matières premières contenant de l'amidon, caractérisé en ce qu'on introduit coaxialement au centre d'un homogéniseur de l'amidon ou des matières premières contenant de l'amidon, sous une forme convenablement broyée, avec des enzymes qui sont appropriées pour l'obtention de l'alcool et avec un apport de vapeur simultané et on les gélifie brusquement, mécaniquement et thermiquement, sous l'effet de l'enzyme finement distribuée à des températures qui ne sont pas inférieures à 50°C et pas supérieures à 115°C, on dégrade la colle d'amidon jusqu'au glucose après la fluidification et on fait fermenter le moût ainsi obtenu de manière telle que la vinasse obtenue avec une substance sèche réduite, après avoir éliminé seulement les impuretés grossières par, par exemple, centrifugation, soit renvoyée plusieurs fois sans autre purification additionnelle et réemployée comme liqueur de traitement jusqu'à ce que la proportion de substance sèche dans la vinasse atteigne 30% de substance sèche.

2. Procédé selon la revendication 1, caractérisé en ce qu'on gélifie à des températures entre 85°C et 95°C.